# EUROPÄISCHE PATENTSCHRIFT

(11) **EP 2 846 733 B1**
(45) Veröffentlichungstag und Bekanntmachung des Hinweises auf die Patenterteilung: **31.08.2016**
(21) Anmeldenummer: 13722719.5
(22) Anmeldetag: 03.05.2013
(51) Int. Cl.: A61F 2/07

(54) **ABDOMINALE BIFURKATIONSPROTHESE**
ABDOMINAL BIFURCATION PROSTHESIS
PROTHÈSE DE BIFURCATION ABDOMINALE

(30) Priorität: 07.05.2012 DE 102012103986
(43) Veröffentlichungstag der Anmeldung: 18.03.2015
(73) Patentinhaber: JOTEC GmbH, 72379 Hechingen (DE)
(72) Erfinder: BARTHOLD, Franz-Peter, 72336 Balingen (DE); WÖRNE, Christian, 73760 Ostfildern (DE)
(74) Vertreter: Witte, Weller & Partner Patentanwälte mbB
(86) Internationale Anmeldenummer: PCT/EP2013/059272
(87) Internationale Veröffentlichungsnummer: WO 2013/167491

(56) Entgegenhaltungen:
- WO-A1-2009/056644
- US-A1- 2005 131 517
- US-A1- 2010 161 025

## Beschreibung

Die vorliegende Erfindung betrifft eine intraluminale Gefäßprothese zur Implantation in ein Blutgefäß, mit einem hohlzylindrischen Grundkörper mit einer proximalen Öffnung mit einem proximalen Durchmesser und einer distalen Öffnung mit einem distalen Durchmesser, wobei die intraluminale Gefäßprothese zumindest einen ersten und einen zweiten Prothesenabschnitt mit in Längsrichtung der Gefäßprothese in einem Abstand hintereinander angeordneten Ringen aus mäanderförmig umlaufenden Stützen und einem an den Ringen befestigtes und diese verbindendes Prothesenmaterial mit einer Gewebestruktur aus einer Vielzahl von miteinander verwebten Kettfäden und Schussfäden aufweist, wobei der erste Prothesenabschnitt einen ersten Durchmesser und der zweite Prothesenabschnitt einen zweiten Durchmesser aufweist, wobei der zweite Durchmesser kleiner als der erste Durchmesser ist.

Insbesondere betrifft die vorliegende Erfindung eine intraluminale Gefäßprothese, die zur Einbringung in die Aorten-Bifurkation ausgebildet ist, und insbesondere eine Aorto-mono-iliacale Gefäßprothese.

Allgemein ist es bekannt, intraluminale Gefäßprothesen, die auch als endovaskuläre Gefäßstents/-stentgrafts bezeichnet werden, zur Behandlung von Aneurysmen in Arterien zu implantieren. Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen oder, wie bei dem sog. falschen Aneurysma bzw. der sog. Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einem Ruptur der Arterie führen mit der Folge, dass der Patient innerlich verblutet.

Zur Behandlung von Aneurysmen ist es bereits bekannt, die betroffene Arterie durch Implantation eines Stents/Stentgrafts zu stabilisieren, um eine Ruptur des Gefäßes zu vermeiden.

Die zur Behandlung von Aneurysmen verwendeten Stents/Stengrafts bestehen dabei im Allgemeinen aus einem röhrchenförmigen Metallrahmen, dessen Mantelfläche mit einer Textil- oder Polymerfolie abgedeckt ist, so dass sich ein hohlzylindrischer Körper ergibt. Zur Implantation wird der Stent - bspw. mittels einer diesen umgebenden und komprimierenden Hülle- radial zusammengedrückt, so dass sich seine Querschnittsfläche deutlich verringert. Der Stent/Stentgraft wird dann mit Hilfe eines Einführsystems in den Bereich des Aneurysmas gebracht, wo der Stent freigesetzt wird. Aufgrund der Federwirkung des Metallrahmens expandiert der Stent wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch den Stent/Stentgraft, wodurch eine weitere Belastung der Aussackung verhindert wird.

Die Expansion des Metallrahmens kann einerseits durch die Verwendung von selbstexpandierendem Metall, wie bspw. Nitinol, bewirkt werden, oder aber durch Einsatz eines Dilatations-Ballons, der von innen in den Metallrahmen eingeführt wird und durch dessen Dilatation auch der Metallrahmen expandiert wird.

Oftmals zweigen an der Stelle des Gefäßes, an welcher ein solcher Stent/Stentgraft bzw. eine solche Gefäßprothese eingeführt werden soll, Seiten-Blutgefäße ab, weshalb bei Einbringung des Gefäßimplantats dann die Gefahr besteht, dass diese Seitengefäße durch die Gefäßprothese im Hauptgefäß bzw. durch das blutdichte Prothesenmaterial von der Blutzufuhr abgeschnitten werden. Daher weisen Gefäßprothesen in diesen Bereichen oftmals Öffnungen, sog. "Fenestrierungen", im Mantel- bzw. Prothesenmaterial auf, um das durch die Gefäßprothese fließende Blut durch diese Öffnungen auch in die vom Gefäß abzweigenden Seiten-Gefäße zu lenken. Dadurch wird auch eine Blutversorgung der Körperbereiche, die von den Seite-Gefäßen versorgt werden, gewährleistete.

Die an solchen Abzweigungs-Bereichen einzubringende Gefäßimplantate weisen in vielen Fällen selber noch vom Gefäßprothesen-Grundkörper abzweigende Seitenäste auf, die in de Seiten-Gefäße hineinreichen. Dadurch wird zusätzlich sicher gestellt, dass auch die Seiten-Gefäße mit Blut versorgt werden.

Insbesondere im Bereich der Aorten-Bifurkation ist es notwendig, die Versorgung der sich in die beiden Arteriae iliacae communes zu gewährleisten, wenn die Aorta in diesem Bereich bspw. aufgrund eines Aneurysmas behandelt werden muss. Im Stand der Technik werden gegenwärtig zur Behandlung von abdominalen Aortenaneurysmen (AAA) üblicherweise Stents bzw. Stengrafts eingesetzt, die aus einem Grundkörper mit zwei sich nach distal erstreckenden Beinchen, welche zur Platzierung in den Arteriae iliacae communes vorgesehen sind; eine solche Prothese wird aufgrund ihrer Form auch als Y-Prothese bezeichnet. Bei vielen dieser Prothesen wird das zweite Beinchen, das auch als kontralaterales Bein bezeichnet wird, separat zum Grundkörper und erst nach dessen Platzierung ein geführt, um insgesamt ein einfacheres Platzieren der Prothese zu ermöglichen. Entsprechend wird bei diesen Prothesen zuerst der Grundköper (oder "Mainbody") über einen - in der Regel - transfemoralen Zugang oberhalb der Aorten-Bifurkation abgesetzt. In einem zweiten Schritt wird dann ein weiteres Implantat, also das kontralaterale Bein, an den Grundkörper angedockt und die Bifurkationsprothese dadurch komplettiert.

Die US 2010/0161025 A1 offenbart eine unverzweigte implantierbare Gefäßprothese mit hintereinander angeordneten Stentringen sowie Prothesenmaterial, das diese verbindet. Das Prothesenmaterial weist eine Region mit reduzierter Dichte auf, die für eine in-situ Fenestrierung vorgesehen ist.

Die WO 2009/056644 A1 offenbart eine Vorrichtung und ein Verfahren zur Lokalisierung von in-situ Fenestrierungen in Gefäßprothesen, wobei die Vorrichtung einen ersten magnetischen Abschnitt und einen Katheter mit einem zweiten magnetischen Abschnitt umfasst.

Schließlich offenbart die US 2005/0131517 A1 eine implantierbare Gefäßprothese mit einem röhrchenförmigen Körper aus hintereinander angeordneten Stentringen, die über ein Prothesenmaterial miteinander verbunden sind. Im Prothesenmaterial sind Fenestrierungen vorgesehen, in welche Seiten-Gefäßprothesen eingebracht werden können.

Ein Nachteil bei den gegenwärtig auf dem Markt erhältlichen Produkten besteht darin, dass mit einem zweiten Führungsdraht die Öffnung im Grundkörper für das kontralaterale Bein sondiert werden muss, um das Einführsystem für das kontralaterale Bein an die richtige Position zu bringen. Das Sondieren wird dabei durch die Zwei-Dimensionalität der bildgebenden Verfahren, die bei diesem Eingriff üblicherweise eingesetzt werden, sowie durch die geometrische Anordnung der Implantate erschwert.

Vor diesem Hintergrund ist Aufgage der vorliegenden Erfindung, eine intraluminale Gefäßprothese, insbesondere für die Implantation im Bereich der Aorten-Bifurkation, bereitzustellen, mit welcher das Sondierungsverfahren vereinfacht und verkürzt werden kann.

Erfindungsgemäß wird diese Aufgabe gelöst durch eine intraluminale Gefäßprothese zur Implantation in ein Blutgefäß, wie in den Ansprüchen definiert, gelöst, mit einem hohlzylindrischen Grundkörper mit einer proximalen Öffnung mit einem proximalen Durchmesser r und einer distalen Öffnung mit einem distalen Durchmessers, wobei die intraluminale Gefäßprothese zumindest einen ersten und einen zweiten Prothesenabschnitt mit in Längsrichtung der Gefäßprothese in einem Abstand hintereinander angeordneten Ringen aus mäanderförmig umlaufenden Stützen und einem an den Ringen befestigtes und diese verbindendes Prothesenmaterial mit einer Gewebestruktur aus einer Vielzahl von miteinander verwebten Kettfäden und Schussfäden aufweist, wobei der erste Prothesenabschnitt einen ersten Durchmesser x und der zweite Prothesenabschnitt einen zweiten Durchmesser y aufweist, wobei der zweite Durchmesser y kleiner als der erste Durchmesser x ist. Ferner weist die erfindungsgemäße intraluminale Gefäßprothese zwischen dem ersten und dem zweiten Prothesenabschnitt einen Übergangsabschnitt auf, der ausschließlich aus Prothesenmaterial mit einer Gewebestruktur gebildet ist und dessen Durchmesser sich von proximal nach distal kontinuierlich verkleinert, wobei das Prothesenmaterial des Übergangsabschnitts zumindest teilweise eine zu dem Prothesenmaterial des ersten und zweiten Prothesenabschnitts unterschiedliche Gewebestruktur mit gitterartig an- und/oder aufgebrachten Verstärkungselementen aufweist.

Mit der erfindungsgemäßen Gefäßprothese wird das Problem der zeitaufwändigen Sondierung gelöst: Da die erfindungsgemäße Gefäßprothese einen Übergangsabschnitt aufweist, dessen Form der abnehmenden Größe der sich verzweigenden Aorta angepasst ist, und da das Prothesenmaterial des Übergangsabschnitt eine andere Gewebestruktur aufweist als das Prothesenmaterial der anderen Prothesenabschnitte, genügt ein einfaches Vorschieben eines Penetrierungsmittels, bspw. eines Penetrationsdrahtes durch das Prothesenmaterial des Übergangsabschnittes, um in diesem Bereich eine Öffnung zu schaffen, in welche das kontralaterale Bein/ die kontralaterale Gefäßprothese eingeführt werden kann. Dabei kann das Penetrierungsmittel den Übergangsabschnitt bzw. dessen Prothesenmaterial an einer beliebigen Stelle durchdringen, und anschließend kann die Spitze des Einführsystems für die kontralaterale Gefäßprothese einfach durch diese Öffnung geführt werden.

Die Gewebestruktur des Übergangsabschnitts weist dabei erfindungsgemäß gitterartig an- und/oder aufgebrachte Verstärkungselemente auf, die gemäß einer bevorzugten Ausführungsform bspw. durch in das Prothesenmaterial eingewebte Textilfäden und/oder aufgebrachte Klebstoffe bewirkt sein können. Die Verstärkungselemente sind dabei nur in der Gewebestruktur des Übergangsabschnitts vorgesehen, nicht außerhalb derer.

Unter "gitterartig angebrachten Verstärkungselementen" wird vorliegend jede Struktur verstanden, mittels derer ein Muster gebildet werden kann, bei welchem sich längliche Elemente kreuzen und dadurch Überschneidungspunkte der länglichen Elemente sowie dazwischen liegende offene Bereiche gebildet werden. Diese Verstärkungselemente sind zusätzlich zu den bereits die Maschenstruktur des Prothesenmaterials bildenden Textilfäden in und/oder auf das Prothesenmaterial eingearbeitet.

Erfindungsgemäß bilden diese gitterartigen oder netzartigen Verstärkungselemente eine Ausrissgrenze, mit der verhindert wird, dass die durch die Verdrängung der Fäden gebildeten größeren Maschen bzw. Öffnungen nicht ausreißen, sondern in einer durch die Gittergröße vorgegebenen Dimension bzw. Grenze verbleiben.

Vorteilhafterweise wird also im Übergangsabschnitt, wo mittels eines entsprechenden Penetrierungsmittels ein Loch/eine Öffnung geschaffen wird, ein weiteres Aus- bzw. Aufreißen der Gewebestruktur bzw. des Prothesenmaterials durch das Gitter bzw. das Netz der Verstärkungselemente verhindert: Die Öffnung bzw. das Loch im Prothesenmaterial wird in einer Gitter-Öffnung eines Gitterelements geschaffen und die Gittergrenzen der Gitterelemente verhindern, dass sich dieses weiter aufreißt. Dadurch wird vermieden, dass sich zu große Öffnungen/Löcher oder Fenster bilden, wodurch wiederum ein sicheres Verankern einer über die geschaffene Öffnung einzuführenden Seitenprothese gewährleistet wird. Der Durchmesser zu bildenden Öffnung hängt dabei vom Durchmesser des über die Öffnung einzuführenden kontralateralen Beins ab, das in der Regel ein Stent oder ein Stentgraft ist, und kann zwischen 2 bis 15 mm betragen.

Der Übergangsabschnitt bzw. Übergangsabschnitt weist dabei einen Durchmesser auf, der sich von proximal an distal kontinuierlich verkleinert; vorzugsweise hat dabei der Übergangsabschnitt ein proximales und ein distales Ende, wobei das proximale Ende an den proximalen Prothesenabschnitt angrenzt und das distale Ende an den distalen Prothesenabschnitt. Weiter vorzugsweise weist der Übergangsabschnitt an seinem proximalen Ende einen Durchmesser auf, der dem Durchmesser des proximalen Prothesenabschnitts entspricht, und das distale Ende des Übergangabschnitts weist einen Durchmesser auf, der dem Durchmesser des distalen Prothesenabschnitts entspricht.

Vorliegend wird mit "proximal" diejenige Position, Richtung oder ein Abschnitt einer Komponente bezeichnet, der am nächsten zum Herzen des zu behandelnden Patienten liegt.

Ferner wird vorliegend mit "distal" diejenige Position, Richtung oder ein Abschnitt einer Komponente bezeichnet, die vom Herzen eine Patienten weiter/am weitesten entfernt ist/führt.

Entsprechend sind vorliegend die "proximale" und die "distale" Öffnung der Gefäßprothese die Öffnungen, durch die hindurch der Blutfluss durch den hohlzylindrischen Körper der Gefäßprothese gewährleistet wird: Wenn die erfindungsgemäße Gefäßprothese in einem Blutgefäß wie bspw. der Aorta implantiert ist, fließt also das vom Herzen kommende Blut durch die proximale Öffnung der Gefäßprothese, und verlässt die Gefäßprothese durch deren distale Öffnung, die in einer der Arteriae iliacae communes lokalisiert ist.

Mit "Stent" wird vorliegend eine Vorrichtung oder eine Struktur bezeichnet, das einer Prothese eine Expansionskraft und/oder eine stützende Funktion verleiht.

Vorliegend bezeichnet der Ausdruck "Stentgraft" eine Prothese die einen Stent sowie ein damit verbundenes Prothesen("Graft")-Material aufweist, das ein Lumen durch zumindest einen Abschnitt der Prothese bildet.

Gemäß einer bevorzugten Ausführungsform weist die erfindungsgemäße intraluminale Gefäßprothese ferner einen dem ersten proximalen Prothesenabschnitt vorgeschalteten Fixierungsabschnitt auf.

Vorteilhafterweise stellt dieser Fixierungsabschnitt eine Fixierungsstruktur dar, mit der die erfindungsgemäße Gefäßprothese proximal in der Aorta fixiert werden kann. Diese Fixierungsstruktur kann dabei bspw. ein ungecoverter Stentabschnitt mit einer bestimmten Struktur oder Anordnung der Stentringe sein.

Gemäß einer weitere bevorzugten Ausführungsform ist die erfindungsgemäße Gefäßprothese zur Implantation in der Aorten-Bifurkation ausgebildet, wobei der ersten Prothesenabschnitt zur Einbringung in die Aorta und der zweite Prothesenabschnitt zur Einbringung in eine der beiden Arteriae iliacae communes ausgebildet ist.

Wie weiter oben erwähnt kann die erfindungsgemäße Gefäßprothese vorteilhafterweise im Bereich der Aorten-Bifurkation eingesetzt werden. Bei diesem Einsatz bildet der erste, proximale Prothesenabschnitt - ggf. zusammen mit einem geeigneten Fixierungsabschnitt - den Teil der Prothese, der in der unverzweigten Aorta platziert wird, mit dem sich nach distal verjüngenden Übergangsabschnitt im Bereich der Bifurkation. Der distale Prothesenabschnitt kommt dann in einer der beiden Arteriae iliacae communes zu liegen. Da die Gefäße Arteriae iliacae communes einen kleineren Durchmesser aufweisen als die Aorta, sollten auch die Prothesenabschnitte, die in den Arteriae iliacae communes platziert werden, einen kleineren Durchmesser aufweisen als die Prothesenabschnitt, die in der Aorta zu liegen kommen.

Entsprechend ist gemäß einer weiteren bevorzugten Ausführungsform der intraluminalen Gefäßprothese bevorzugt, wenn der proximale Durchmesser der proximalen Öffnung dem ersten Durchmesser des ersten Prothesenabschnitts entspricht, und der distale Durchmesser der distalen Öffnung dem zweiten Durchmesser des zweiten Prothesenabschnitts.

Bei dieser Ausführungsform besitzt somit der erste proximale Prothesenabschnitt den Durchmesser, den auch die proximale Öffnung der Gefäßprothese aufweist, mithin also der Öffnung der Gefäßprothese, in die der Blutfluss geleitet ist. Der distale Prothesenabschnitt besitzt vorteilhafterweise den Durchmesser, den die distale Öffnung der Gefäßprothese aufweist, mithin also die Öffnung der Gefäßprothese, über die der Blutstrom die Gefäßprothese wieder verlässt.

Gemäß einer weiteren bevorzugten Ausführungsform der intraluminalen Gefäßprothese weist das Prothesenmaterial im Übergangsabschnitt eine Reißfestigkeit auf, die geringer ist als die Reißfestigkeit des Prothesenmaterials des ersten und zweiten Prothesenabschnitts.

Hierbei wird unter "Reißfestigkeit" die Eigenschaft des Prothesenmaterials verstanden, der Durchdringung bzw. dem Zerreißen der Gewebestruktur mit Durchdringungs-/Penetrierungsmitteln zu widerstehen. Daher ist erfindungsgemäß das Prothesenmaterial im Übergangsabschnitt leichter mit einem entsprechendem Penetrierungsmittel zu zerreißen oder zu durchdringen als im Prothesenmaterial des proximalen und distalen Prothesenabschnitts. Dies bietet den Vorteil, dass der Anwender bei Einführung eines Penetrationsmittels wie bspw. ein Führungsdraht mit der Spitze dieses Mittels/Führungsdrahtes durch das Prothesenmaterial im Übergangsabschnitt leichter vordringen kann und bereits aufgrund der unterschiedlichen Reißfestigkeit erkennen kann, dass der Übergangsabschnitt und damit die korrekte Penetrationsstelle erreicht ist. Die geringere Reißfestigkeit kann bspw. durch Einsatz unterschiedlicher Garne in diesem Bereich erreicht werden, sowie bspw. durch eine geringere Maschendichte der verwobenen Fäden - in der Regel Kettfäden und Schussfäden - oder durch ein nicht-miteinander-Verweben von Kett- und Schussfäden.

In einer anderen bevorzugten Ausführungsform der erfindungsgemäßen Gefäßprothese besteht das Prothesenmaterial aus einer Vielzahl von miteinander verwebten Kettfäden und Schussfäden, und das Prothesenmaterial weist im Übergangbereich eine Gewebestruktur aus zumindest teilweise nicht miteinander verwebten Kettfäden und Schussfäden auf.

Diese Ausführungsform hat den Vorteil, dass durch das nichtmiteinander-Verweben der Kett- und Schussfäden Zonen oder Bereiche entstehen, in denen die Fäden praktisch lediglich übereinander liegen. Der Anwender kann dann bspw. mit Hilfe eines Katheters, eines Dilators oder eines Führungsdrahtes der deren Spitzen, oder eines sonstigen geeigneten Elements, diese Gewebestruktur derart durchdringen, dass das Gewebe nicht zerrissen, zerstört, durchlöchert oder in sonstiger Weise verletzt wird, sondern die Fäden der Gewebestruktur von dem einzusetzenden Element verdrängt werden, so dass sich sozusagen eine große Masche bildet, die die Öffnung oder Fenestrierung für das abgehende Seitengefäß darstellt. Ermöglicht wird dies durch die besondere Gewebestruktur in dem Übergangsabschnitt, nämlich derart, dass die Kettfäden und die Schussfäden, die im übrigen Prothesenmaterial miteinander verwebt sind, im Übergangsabschnitt nicht miteinander verwebt sind. Dies hat zur Folge, dass im Übergangsabschnitt Kettfäden und Schussfäden lediglich übereinander liegen, also praktisch "schichtweise" angeordnet sind. Dies wird durch ein besonderes Webverfahren erreicht, mithilfe dessen erreicht wird, dass gezielt und ausschließlich im Übergangsabschnitt die Fadensysteme - also Kett- und Schussfaden - nicht miteinander verwebt werden.

Mit den Begriffe "Kettfäden" und "Schussfäden" sind vorliegend - wie auch üblicherweise im die Erfindung betreffenden Gebiet - die bei Geweben üblicherweise verwendeten beiden Fadensysteme gemeint; zur manuellen oder maschinellen Herstellung gewebter textiler Erzeugnisse werden mindestens zwei Fadensysteme rechtwinklig oder nahezu rechtwinklig verkreuzt. Dabei werden üblicherweise die Fäden in Längsrichtung als Kette oder Kettfäden bezeichnet, und die Querfäden Schuss oder Schussfäden. Die Fäden werden durch Fadenkreuzung verbunden, was nicht bedeutet, dass die Fäden kreuzend aufeinander liegen, sondern dass Fäden in einem bestimmten Rhythmus über und unter den querliegenden Fäden durchgeführt werden.

Üblicherweise werden die Garne, die zur Herstellung von intraluminalen Gefäßprothesen eingesetzt werden, in verschiedenen Richtungen zusammengewebt, bspw. derart, dass ein Satz Kettenfäden in Längsrichtung parallel zu den Webkanten verläuft und dadurch die Breite des gewebten Produktes darstellt, und dass ein Satz Schussfäden dann von Webkante zu Webkante unter einem rechten Winkel zu den Kettenfäden in diese gewebt werden.

Die hohlzylindrische Form der Gefäßprothese wird dann üblicherweise dadurch erreicht, dass das gewebte Prothesenmaterial vernäht und ggf. zugeschnitten wird. So werden bspw. auch die mäanderförmig umlaufenden Ringe jeweils mit dem Prothesenmaterial vernäht, zur Ausbildung der Röhrenform der Prothese.

Durch die im Übergangsabschnitt nicht miteinander verwebten Kett- und Schussfäden wird in diesen Bereichen eine Struktur geschaffen, die leicht mit einem Erweiterungselement - von innen oder von außen der Gefäßprothese - durchdrungen werden kann, wobei beim Durchdringen lediglich die Fäden in diesem Bereich durch das Erweiterungselement verdrängt werden und die Fäden nicht zerrissen oder Durchschnitten werden. Eine Verdrängung der Fäden zur Ausbildung einer Öffnung bzw. Fenestrierung ist in den anderen, nicht mit einem solchen erfindungsgemäßen Übergangsabschnitt versehenen Prothesenmaterial-Bereichen nicht möglich, da dort die Gewebestruktur durch die Verwebung der Fadensysteme zu dicht ist.

Vorteilhafterweise kann die Gewebestruktur des Prothesenmaterials im Übergangsabschnitt durch eine integrierte Webung hergestellt und das Prothesenmaterial einstückig gewebt und ausgebildet werden. Hierzu ist der Webeprozess derart ausgebildet bzw. mittels entsprechender Software programmiert, dass im Übergangsabschnitt Kettfäden und Schussfäden bzw. Kettfaden und Schussfaden nicht miteinander verwebt werden.

Vorliegend werden die Begriffe "Öffnung" und "Fenestrierung" auch synonym verwendet.

Entsprechend weist gemäß einer weiteren bevorzugten Ausführungsform der erfindungsgemäßen intraluminalen Gefäßprothese diese femer zumindest einen Stent oder Stentgraft auf, der zur Einbringung in ein von dem Gefäß, insbesondere der Aorta, abzweigendes Gefäß, insbesondere die Arteria iliaca communis, einbringbar und mit der Gefäßprothese über deren Übergangsabschnitt verbindbar ausgebildet ist.

Diese Ausführungsform umfasst damit neben dem Haupt-Körper der Gefäßprothese auch zumindest einen Stent oder Stentgraft, der in das abzweigende Gefäß platziert und im Übergangsabschnitt der intraluminalen Gefäßprothese bspw. über einen sogenannten Ankerkranz, also durch sich nach außen und von der Längsachse des Stents/Stentgraft weg erstreckende/expandierende Fixierelemente, wie Stentringe, verankert wird.

Die erfindungsgemäße Gefäßprothese kann in einer Weiterbildung ferner entweder am hohlzylindrischen Grundkörper, insbesondere im Übergangsabschnitt, und/oder an dem Seitenast/den Seitenästen bspw. röntgendichte Marker aufweisen, die das Einführen und Positionieren der Gefäßprothese erleichtern.

Erfindungsgemäß ist ferner ein Verfahren zur Einbringung einer erfindungsgemäßen intraluminalen Gefäßprothese wie oben beschrieben offenbart, uns zwar in die Aorten-Bifurkation eines Patienten, mit den folgenden Schritten:
- Einbringen und Freisetzen der intraluminalen Gefäßprothese eines Patienten, derart, dass der erste Prothesenabschnitt in der Aorta und der zweite Prothesenabschnitt in einer der beiden Arteriae iliacae communes platziert wird,
- Ausbildung von zumindest einer Öffnung im Übergangsabschnitt der Gefäßprothese durch Zerreißen und/oder Verdrängen des Prothesenmaterials im Übergangsabschnitt und
- Einbringen eines Stentgrafts durch die Öffnung im Übergangsabschnitt der Gefäßprothese zur Ausbildung von zumindest einem Seitenast der intraluminalen Gefäßprothese in der anderen Arteria iliaca communis.

Die zumindest eine Öffnung kann mittels eines geeigneten Erweiterungs-/Penetrierungselements geschaffen werden, wie bspw. über einen Führungsdraht oder einen Katheter. Die Gewebestruktur, die im Übergangsabschnitt eine geringere Reißfestigkeit besitzt, kann damit einfach durchstoßen oder das Prothesenmaterial kann einfach verdrängt werden, zur Ausbildung der Öffnung. Durch die ggf. vorliegenden zusätzlichen Verstärkungselemente wird das Prothesenmaterial nicht weiter aufreißen, weshalb die Gefahr eines Einreißens des Prothesenmaterials in Bereiche außerhalb des Übergangsabschnittes vermieden wird.

Dabei wird vorliegend unter einem "Erweiterungselement" jedes Mittel verstanden, mit Hilfe dessen in dem Übergangsabschnitt eine Öffnung/Fenestrierung geschaffen werden kann. Geeignete Erweiterungselemente sind dabei bspw. Ein Katheter, Dilator oder ein Führungsdraht, bzw. die Katheter-, Dilatoren- oder Führungsdrahtspitze.

Katheter, Dilatoren und Führungsdrähte sind im Stand der Technik hinreichend bekannt und werden bereits seit längerem im Zusammenhang mit Gefäßprothesen eingesetzt, so dass dem Fachmann klar sein wird, welche Mittel er erfindungsgemäß einsetzen kann.

Mit diesem Verfahren kann erstmals die Möglichkeit geschaffen werden, eine Gefäßprothese in ein Gefäß, insbesondere in die Aorten-Bifurkation, zum Zwecke der Ausbildung von zumindest einer Fenestrierung/Öffnung einzuführen, ohne dass die genaue Rotations-Position der Gefäßprothese in dem Gefäß berücksichtigt werden müsste. Der chirurgische Eingriff wird dadurch wesentlich erleichtert und beschleunigt.

Typische Anwendungsgebiete sind, wie bereits weiter oben beschrieben, der Einsatz im Aortenbogen, im thorakalen und im iliakalen Bereich.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung eines Ausschnitts einer Ausführungsform der erfindungsgemäßen intraluminalen Gefäßprothese;
- Fig. 2: eine schematische Darstellung einer Ausführungsform eines Seitenasts der erfindungsgemäßen Gefäßprothese;
- Fig.3: eine schematische Darstellung der in Fig. 1 gezeigten, in die Aorten-Bifurkation eingebrachten Ausführungsform der erfindungsgemäßen Gefäßprothese; und
- Fig. 4: die in Fig. 3 gezeigte, in die Aorten-Bifurkation eingebrachte Ausführungsform, mit eingeführtem Seitenast ("kontralaterales Bein").

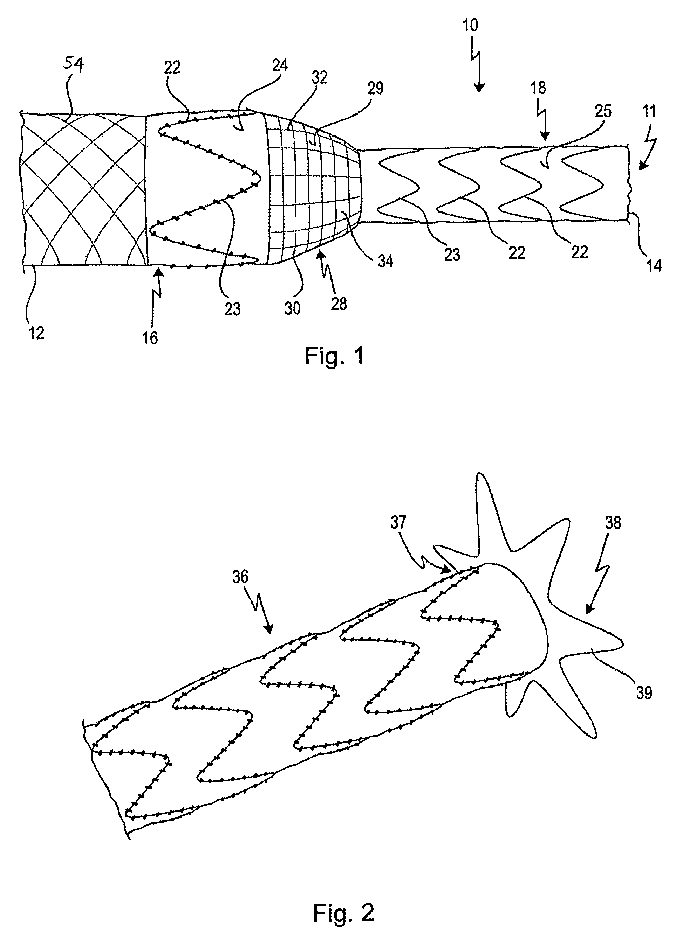

In Fig. 1 ist mit 10 insgesamt eine Ausführungsform einer erfindungsgemäßen Gefäßprothese mit einem hohlzylindrischen Körper 11 gezeigt. Der hohlzylindrische Köper 11 weist eine proximale Öffnung 12 und eine distale Öffnung 14 auf, wobei die distale Öffnung 12 einen Durchmesser r besitzt, der größer ist als der Durchmesser s der distalen Öffnung 14.

Die in Fig. 1 gezeigte Gefäßprothese 10 weist fernern einen ersten, proximalen Prothesenabschnitt 16 sowie einen zweiten distalen Prothesenabschnitt 18 auf. Beide Prothesenabschnitte 16, 18 weisen in Längsrichtung der Gefäßprothese 10 in einem Abstand hintereinander angeordnete Ringe 22 aus mäanderförmig umlaufenden Stützen 23 und ein an den Ringen 22 befestigtes und diese verbindenden Prothesenmaterial 24, 25 auf.

Der erste, proximale Prothesenabschnitt 16 weist einen Durchmesser x und der zweite, distale Prothesenabschnitt 18 einen zweiten Durchmesser y.

Das Prothesenmaterial 24, 25 der Prothesenabschnitte 16, 18 besitzt eine Gewebestruktur 26 aus einer Vielzahl von miteinander verwebten Kettfäden und Schussfäden (nicht gezeigt).

Zwischen ersten proximalen und zweiten distalen Prothesenabschnitt 16, 18 ist ein Übergangsabschnitt bzw. Übergangsabschnitt 28 vorgesehen, der keine Ringe 22 aus mäanderförmig umlaufenden Stützen aufweist, sondern nur bzw. ausschließlich aus Prothesenmaterial 29 gebildet ist, das wiederum eine Gewebestruktur 30 aufweist. Diese Gewebestruktur 30 des Übergangsabschnitts 28 ist zu den Gewebestrukturen des Prothesenmaterials 24, 25 der Prothesenabschnitte 16, 18 unterschiedlich. In der gezeigten Ausführungsform hat die Gewebestruktur 30 im Übergangsabschnitt 28 eine geringere Reißfestigkeit als die Gewebestruktur des Prothesenmaterials 24, 25 der Prothesenabschnitte 16, 18. Ferner weist die Gewebestruktur 30 Verstärkungselemente 32 auf, die in/auf die Gewebestruktur 30 bzw. das Prothesenmaterial 29 gitterartig an-/aufgebracht sind. Die Verstärkungselemente 32 können dabei - zusätzlich zu den die Gewebestruktur 30 bildenden Textilfäden - in diese, also die Gewebestruktur eingebrachte Textilfäden sein, oder auf die Gewebestruktur 30 bzw. das Prothesenmaterial 29 aufgebrachte Klebstofffäden. Diese Verstärkungselemente 32 bilden eine gitterartige Struktur im Übergangsabschnitt, mit Gitteröffnungen 33 und Begrenzungselementen 34 der Gitteröffnungen 33.

Fig. 1 ist ferner zu entnehmen, dass der Übergangsabschnitt 28 einen Durchmesser aufweist, der sich kontinuierlich von proximal nach distal verkleinert. An seinem proximalen Ende 50 weist der Übergangsabschnitt 28 einen Durchmesser auf, der dem Durchmesser des ersten Durchmesser x des ersten, proximalen Prothesenabschnitt entspricht, und an seinem distalen Ende 51 einen Durchmesser, der dem Durchmesser des zweiten Durchmessers des zweiten, distalen Prothesenabschnitts entspricht.

Die Gefäßprothese 10 weist ferner einen Fixierungsabschnitt 54 auf, über welchen sie in einem Gefäß proximal verankert wird. Dieser besteht üblicherweise aus Stentringen, die sich nach deren Expansion an die Gefäßwand anlagern und die Gefäßprothese dort fixieren.

Zur Bildung einer Öffnung 49 für einen Seitenast der Gefäßprothese 10 kann mittels eines Penetrierungsmittels, bspw. einem Katheter, einem Führungsdraht oder einem Dilator, die Gewebestruktur 30 im Übergangsabschnitt 28 penetriert werden, wobei die Öffnung 49 in einer Gitteröffnung 33 gebildet wird. Die Begrenzungselemente 34 der Gitteröffnungen 33 gewährleisten dabei, dass die in einer Gitteröffnung 33 gebildete Öffnung 49 für den Seitenast, bzw. das kontralaterale Bein, nicht weiter aufreißt und begrenzen daher die Öffnung 49.

Fig. 2 zeigt ein Beispiel für ein kontralaterales Bein bzw. einen Seitenast 36, das/der in die Öffnung 49 in dem Übergangsabschnitt 28 zur Ausbildung eines Seitenasts 36 der Gefäßprothese 10 geführt wird. Das Bein/der Seitenast 36 weist an seinem Ende 37, mit dem es/er in der Gefäßprothese 10 verankert wird, einen Ankerkranz 38 auf. Dieser ist durch von der Längsachse des Beines 36 nach außen zeigenden und expandierenden Stentringen 39 gebildet. Das kontralaterale Bein 36 besitzt einen hohlzylindrischen Grundkörper 37, der wie die Gefäßprothese 10 in Längsrichtung und in einem Abstand hintereinander angeordnete Ringe 38 aus mäanderförmig umlaufenden Stützen aufweist sowie ein an den Ringen befestigtes und diese verbindendes Prothesenmaterial 39.

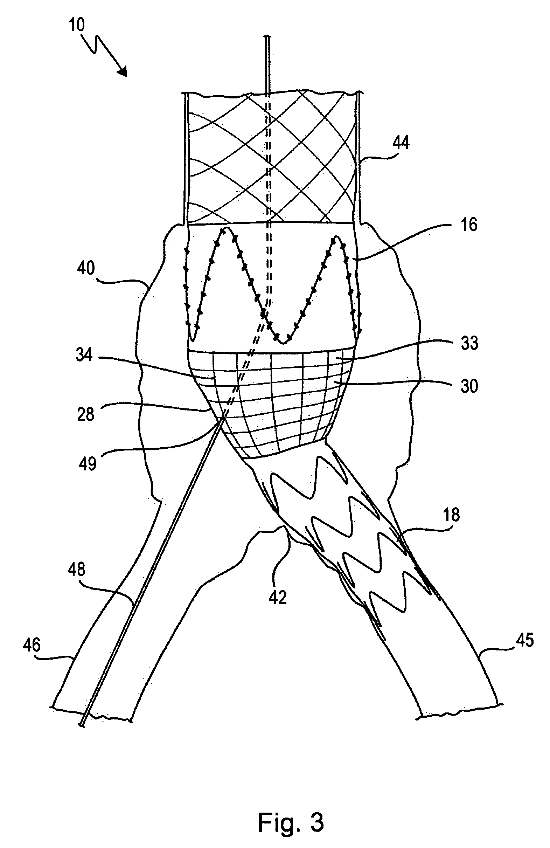

Fig. 3 zeigt eine schematische Darstellung der Gefäßprothese aus Fig. 1, die ein Aneurysma 40 im Bereich der Aorten-Bifurkation 42 überspannt. Der Übergangsabschnitt 28 kommt dabei über der Bifurkation 42 zu liegen, der erste, proximale Prothesenabschnitt 16 in der Aorta 44 und der zweite, distale Prothesenabschnitt 18 in einer der Arteriae iliacae communes 45. Die Gefäßprothese kann über ein übliches Einführsystem (nicht gezeigt) mit einer die Gefäßprothese 10 komprimierenden Hülle und einem Pusher in die Gefäße eingeführt und freigesetzt werden, wie einleitend allgemein beschrieben.

In Fig. 3 ist ferner gezeigt, wie von proximal kommend ein Führungsdraht 48 in die Gefäßprothese 10 eingeführt wurde, und wie dieser das Prothesenmaterial 29 bzw. ein Gitteröffnung 33 der Gewebestruktur 30 zur Bildung der Öffnung 49 in dem Übergangsabschnitt 28 penetriert. Der Führungsdraht 48 wird dabei in die andere, Prothesen-freie Arteria iliaca communis 46 eingeführt.

Nach Legung des Führungsdrahtes 48 wird über diesen und durch die Öffnung 49 im Prothesenmaterial 29 im Übergangsabschnitt 28 das Einführsystem für das kontralaterale Bein 36 geführt und derart freigesetzt, dass der Ankerkranz 38 innerhalb der Gefäßprothese 10 an der Öffnung 49 im Übergangsabschnitt 28 expandiert und den Seitenast/das kontralaterale Bein 36 in dieser verankert. Der restliche Teil des kontralateralen Beins 36 wird in der Arteria iliaca communis 46 freigesetzt, wo sich die Wände des kontralateralen Beins 36 an die Gefäßwand der Arteria iliaca communis 46 legen und diese dort fixieren.

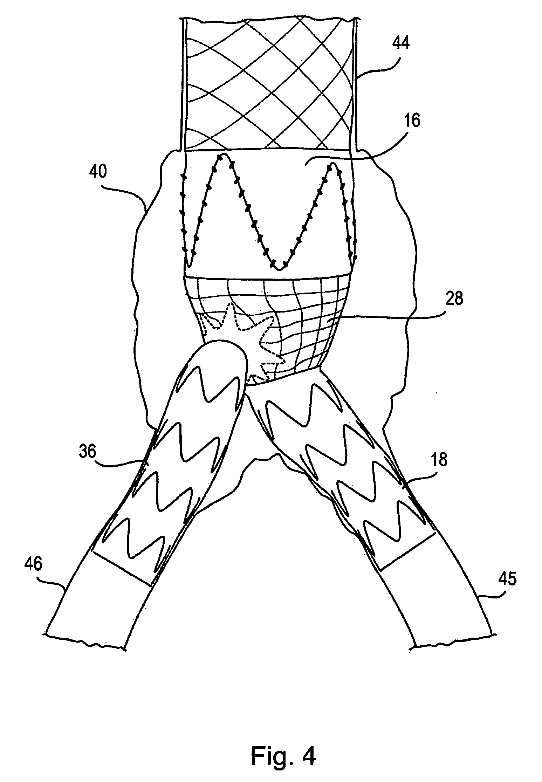

Der letzte Schritt der vollständigen Freisetzung ist in Fig. 4 gezeigt, in der dargestellt ist, wie das kontralaterale Bein 36 in der Gefäßprothese 10 bzw. in deren Übergangsabschnitt 28 verankert ist, sowie in der Arteria iliaca communes fixiert ist. Nach Freisetzung des kontralateralen Beins 36 besitzt die Anordnung aus Gefäßprothese 10 und kontralateralem Bein 36 die Form eines Y.

## Patentansprüche

1. Intraluminale Gefäßprothese (10) zur Implantation in ein Blutgefäß, insbesondere in die Aorta Bifurcatio, mit einem hohlzylindrischen Grundkörper (11) mit einer proximalen Öffnung (12) mit einem proximalen Durchmesser r und einer distalen Öffnung (14) mit einem distalen Durchmesser s, wobei die intraluminale Gefäßprothese (10) zumindest einen proximalen ersten (16) und einen distalen zweiten (18) Prothesenabschnitt mit in Längsrichtung der Gefäßprothese (10) in einem Abstand hintereinander angeordneten Ringen (22) aus mäanderförmig umlaufenden Stützen (23) und einem an den Ringen (22) befestigtes und diese verbindendes Prothesenmaterial (24, 25) mit einer Gewebestruktur (26) aus einer Vielzahl von miteinander verwebten Kettfäden und Schussfäden aufweist, wobei der erste Prothesenabschnitt (16) einen ersten Durchmesser x und der zweite Prothesenabschnitt (18) einen zweiten Durchmesser y aufweist, wobei der zweite Durchmesser y kleiner als der erste Durchmesser x ist,
**dadurch gekennzeichnet, dass** die intraluminale Gefäßprothese (10) zwischen dem ersten (16) und dem zweiten (18) Prothesenabschnitt einen Übergangsabschnitt (28) aufweist, der ausschließlich aus Prothesenmaterial (29) mit einer Gewebestruktur (30) gebildet ist und dessen Durchmesser sich von proximal nach distal kontinuierlich verkleinert, wobei das Prothesenmaterial (29) des Übergangsabschnitts (28) über dessen gesamten Umfang eine zu dem Prothesenmaterial (24, 25) des ersten (16) und zweiten (18) Prothesenabschnitts unterschiedliche Gewebestruktur (26) mit gitterartig an- und/oder aufgebrachten Verstärkungselementen (32) aufweist.

2. Intraluminale Gefäßprothese (10) nach Anspruch 1, **dadurch gekennzeichnet, dass** sie einen dem ersten Prothesenabschnitt (16) in proximaler Richtung vorgeschalteten Fixierungsabschnitt (54) zur proximalen Verankerung der Gefäßprothese (10) im Gefäß aufweist.

3. Intraluminale Gefäßprothese (10) nach Anspruch 1 oder 2, **dadurch gekennzeichnet, dass** sie zur Implantation in der Aorten-Bifurkation (42) ausgebildet ist, wobei der ersten Prothesenabschnitt (16) zur Einbringung in die Aorta (44) und der zweite Prothesenabschnitt (18) zur Einbringung in ein von der Aorta (44) abzweigendes Gefäß, insbesondere der Arteria iliaca communis (45, 46), ausgebildet ist.

4. Intraluminale Gefäßprothese (10) nach einem der Ansprüche 1 bis 3, **dadurch gekennzeichnet, dass** der proximale Durchmesser r der proximalen Öffnung (12) dem ersten Durchmesser x des ersten Prothesenabschnitts (16) entspricht, und der distale Durchmesser s der distalen Öffnung (14) dem zweiten Durchmesser y des zweiten Prothesenabschnitts (18).

5. Intraluminale Gefäßprothese (10) nach einem der Ansprüche 1 bis 4, **dadurch gekennzeichnet, dass** die Gewebestruktur (30) im Übergangsabschnitt (28) eine Reißfestigkeit aufweist, die geringer ist als die Reißfestigkeit der Gewebestruktur des Prothesenmaterials (24, 25) des ersten und zweiten Prothesenabschnitts (16; 18).

6. Intraluminale Gefäßprothese (10) nach einem der Ansprüche 1 bis 5, **dadurch gekennzeichnet, dass** die gitterartig angebrachten Verstärkungselemente (32) durch eingewebte Textilfäden und/oder aufgebrachte Klebstoffe bewirkt sind.

7. Intraluminale Gefäßprothese (10) nach einem der Ansprüche 1 bis 5, **dadurch gekennzeichnet, dass** das Prothesenmaterial (24, 25, 29) aus einer Vielzahl von miteinander verwebten Kettfäden und Schussfäden besteht, und dass das Prothesenmaterial (29) im Übergangbereich (28) eine Gewebestruktur (30) aus zumindest teilweise nicht miteinander verwebten Kettfäden und Schussfäden aufweist.

8. Intraluminale Gefäßprothese (10) nach einem der Ansprüche 1 bis 7, **dadurch gekennzeichnet, dass** sie ferner einen Stent oder Stentgraft (36) umfasst, der zur Einbringung in ein von dem Gefäß abzweigendes Gefäß, insbesondere in die Arteria iliaca communis (46), einbringbar und mit der Gefäßprothese (10) über deren Übergangsabschnitt (28) verbindbar ausgebildet ist.

## Claims

1. Intraluminal vascular prosthesis (10) for implantation into a blood vessel, in particular into the Aorta Bifurcatio, having a hollow cylindrical body (11) comprising a proximal opening (12) with a proximal diameter and a distal opening (14) with a distal diameter s, the intraluminal vascular prosthesis (10) comprising at least a proximal first (16) and a distal second (18) prosthetic section with rings (22) which are disposed and spaced apart successively in longitudinal direction of the vascular prosthesis (10) and which are made up of meandering circumferential supports (23), and with a prosthesis material (24, 25) which is fixed to the rings (22) and which connects them, the prosthesis material (24, 25) having a fabric structure (26) of a plurality of interwoven warp threads and weft threads, wherein the first prosthetic section (16) comprises a first diameter x and the second prosthetic section (18) a second diameter y, the second diameter y being smaller than the first diameter x,
**characterized in that** the intraluminal vascular prosthesis (10), between the first (16) and the second (18) prosthetic section, comprises a transition section (28), which is made up exclusively from prosthetic material (29) having a fabric structure (30), and whose diameter continuously decreases from proximal to distal, wherein the prosthetic material (29) of the transition section (28), along its entire circumference, comprises a fabric structure (26) with reinforcing elements (32) being fixed or applied in a grid-pattern, which fabric structure (26) differs from the prosthetic material (24, 25) of the first (16) and second (18) prosthetic section.

2. The intraluminal vascular prosthesis (10) of claim 1, **characterized in that** it comprises a fixing section (54) for proximal anchoring of the vascular prosthesis (10) in the vessel, the fixing section (54) being positioned, in proximal direction, upstream of the first prosthetic section (16).

3. The intraluminal vascular prosthesis (10) of claim 1 or 2, **characterized in that** it is designed for implantation into the aortic bifurcation (42), with the first prosthetic section (16) being designed for implantation into the aorta (44) and the second prosthetic section (18) being designed for implantation into a vessel branching off the aorta (44), in particular into the Arteria iliaca communis (45, 46).

4. The intraluminal vascular prosthesis (10) of any of claims 1 to 3, **characterized in that** the proximal diameter r of the proximal opening (12) corresponds to the first diameter x of the first prosthetic section (16), and the distal diameter s of the distal opening (14) corresponds to the second diameter y of the second prosthetic section (18).

5. The intraluminal vascular prosthesis (10) of any of claims 1 to 4, **characterized in that** the fabric structure (30) in the transition section (28) has a tensile strength which is smaller than the tensile strength of the fabric structure of the prosthetic material (24, 25) of the first and second prosthetic sections (16; 18).

6. The intraluminal vascular prosthesis (10) of any of claims 1 to 5, **characterized in that** the grid-patterned reinforcing elements (32) are effected by interwoven textile threads and/or deposited adhesives.

7. The intraluminal vascular prosthesis (10) of any of claims 1 to 5, **characterized in that** the prosthetic material (24, 25, 29) consists of a plurality of interwoven warp threads and weft threads, and that the prosthetic material (29), in the transition section (28), comprises a fabric structure (30) having warp threads and weft threads which, at least partially, are not interwoven.

8. The intraluminal vascular prosthesis (10) of any of claims 1 to 7, **characterized in that** it further comprises a stent or stent graft (36), the stent or stent graft (36) being designed such, that it is implantable into a vessel branching off the vessel, in particular into the Arteria iliaca communis (46), and that it is connectable with the vascular prosthesis (10) via the vascular prosthesis' transition section (28).

## Revendications

1. Prothèse vasculaire intraluminale (10) pour implantation dans un vaisseau sanguin, en particulier dans la Aorta Bifurcatio, avec un corps de base cylindrique creux (11) avec une ouverture proximale (12) présentant un diamètre proximal ret une ouverture distale (14) présentant un diamètre distal s, dans laquelle la prothèse vasculaire intraluminale (10) présente une première section de prothèse proximale (16) et une deuxième section de prothèse distale (18) avec des anneaux (22) composés de tuyaux (23) au pourtour sinueux disposés à une distance l'un derrière l'autre dans la direction longitudinale de la prothèse vasculaire (10) et avec un matériau de prothèse (24, 25) fixé aux anneaux (22) et assemblant ceux-ci, qui présente une structure de tissu (26) en une multiplicité de fils de chaîne et de fils de trame tissés les uns avec les autres, dans laquelle la première section de prothèse (16) présente un premier diamètre x et la deuxième section de prothèse (18) présente un deuxième diamètre y, dans laquelle le deuxième diamètre y est plus petit que le premier diamètre x,
**caractérisée en ce que** la prothèse vasculaire intraluminale (10) présente entre la première section de prothèse (16) et la deuxième section de prothèse (18) une section de transition (28), qui est formée exclusivement de matériau de prothèse (29) avec une structure de tissu (30) et dont le diamètre diminue en continu de l'extrémité proximale à l'extrémité distale, dans laquelle le matériau de prothèse (29) de la section de transition (28) présente sur toute sa périphérie une structure de tissu (26) différente du matériau de prothèse (24, 25) de la première (16) et de la deuxième (18) sections de prothèse, avec des éléments de renforcement (32) appliqués et/ou déposés en forme de treillis.

2. Prothèse vasculaire intraluminale (10) selon la revendication 1, **caractérisée en ce qu'**elle présente une section de fixation (54) connectée en amont de la première section de prothèse (16) en direction proximale pour l'ancrage proximal de la prothèse vasculaire (10) dans le vaisseau.

3. Prothèse vasculaire intraluminale (10) selon la revendication 1 ou 2, **caractérisée en ce qu'**elle est conçue pour l'implantation dans la bifurcation de l'aorte (42), dans laquelle la première section de prothèse (16) est conçue pour l'introduction dans l'aorte (44) et la deuxième section de prothèse (18) est conçue pour l'introduction dans un vaisseau partant de l'aorte (44), en particulier dans l'Arteria iliaca communis (45, 46).

4. Prothèse vasculaire intraluminale (10) selon l'une quelconque des revendications 1 à 3, **caractérisée en ce que** le diamètre proximal r de l'ouverture proximale (12) correspond au premier diamètre x de la première section de prothèse (16) et le diamètre distal s de l'ouverture distale (14) correspond au deuxième diamètre y de la deuxième section de prothèse (18).

5. Prothèse vasculaire intraluminale (10) selon l'une quelconque des revendications 1 à 4, **caractérisée en ce que** la structure de tissu (30) dans la section de transition (28) présente une résistance à la déchirure, qui est plus faible que la résistance à la déchirure de la structure de tissu du matériau de prothèse (24, 25) de la première et de la deuxième sections de prothèse (16, 18).

6. Prothèse vasculaire intraluminale (10) selon l'une quelconque des revendications 1 à 5, **caractérisée en ce que** les éléments de renforcement (32) appliqués en forme de treillis sont créés par des fils textiles incorporés et/ou par des matières adhésives déposées.

7. Prothèse vasculaire intraluminale (10) selon l'une quelconque des revendications 1 à 5, **caractérisée en ce que** le matériau de prothèse (24, 25, 29) se compose d'une multiplicité de fils de chaîne et de fils de trame tissés les uns avec les autres, et **en ce que** le matériau de prothèse (29) dans la section de transition (28) présente une structure de tissu (30) en fils de chaîne et en fils de trame au moins partiellement non tissés les uns avec les autres.

8. Prothèse vasculaire intraluminale (10) selon l'une quelconque des revendications 1 à 7, **caractérisée en ce qu'**elle comprend en outre un stent ou un stent graft (36), qui est conçu sous une forme pouvant être introduite pour l'introduction dans un vaisseau ramifié du vaisseau, en particulier dans l'Arteria iliaca communis (46) et pouvant être assemblée à la prothèse vasculaire (10) par l'intermédiaire de sa section intermédiaire (28).
